# FASCICULE DE BREVET EUROPEEN

(11) **EP 2 896 428 B1**
(45) Date de publication et mention de la délivrance du brevet: **09.11.2016**
(21) Numéro de dépôt: 14195205.1
(22) Date de dépôt: 27.11.2014
(51) Int. Cl.: A61N 1/39, G06N 3/02

(54) **Ensemble de réseau de neurones pour l'évaluation et l'adaptation d'une thérapie antitachycardique par un défibrillateur implantable**
Einheit eines Neuronennetzes für die Bewertung und Anpassung einer Anti-Tachykardie-Therapie mithilfe eines implantierbaren Defibrillators
Neural network assembly for evaluating and adapting an anti-tachycardia therapy by an implantable defibrillator

(30) Priorité: 16.01.2014 FR 1450357
(43) Date de publication de la demande: 22.07.2015
(73) Titulaire: Sorin CRM SAS, 92143 Clamart Cedex (FR)
(72) Inventeur: Cappelaere, Charles-Henri, 75014 Paris (FR); Amblart, Amel, 92330 Sceaux (FR); Christophle-Boulard, Sylvain, 91700 Sainte Geneviève des Bois (FR); Dreyfus, Gérard, 91190 Gif sur Yvette (FR); Dubois, Rémi, 33700 Mérignac (FR); Roussel, Pierre, 75005 Paris (FR)
(74) Mandataire: Dupuis-Latour, Dominique

(56) Documents cités:
- DE-C1- 4 337 110
- US-A- 5 251 626
- US-A- 5 280 792
- US-A1- 2003 097 153
- US-A1- 2008 147 130
- US-A1- 2013 123 869
- US-B1- 6 263 238
- US-B2- 7 657 313

## Description

L'invention concerne les "dispositifs médicaux implantables actifs" tels que définis par la directive 90/385/CEE du 20 juin 1990 du Conseil des communautés européennes, plus précisément les défibrillateurs automatiques implantables (DAIs ou ICDs, *Implantable Cardioverters-Defibrillators*), qui sont des dispositifs susceptibles d'appliquer une thérapie antitachycardique par délivrance de chocs de défibrillation ou de cardioversion, c'est-à-dire d'impulsions électriques de haute énergie, dépassant notablement l'énergie fournie pour une simple stimulation du myocarde.

Ces dispositifs sont généralement implantés en prévention primaire chez des patients présentant un tableau clinique particulier, typiquement les patients victimes d'un infarctus avec des anomalies électrophysiologiques, ou les patients coronariens et/ou insuffisants cardiaques ayant une fraction d'éjection réduite.

Plus précisément, les recommandations actuelles pour une indication d'implantation prophylactique d'un défibrillateur sont :
- patient coronarien, sans ou avec symptômes d'insuffisance cardiaque légère ou modérée, et fraction d'éjection ≤ 30 % mesurée au moins un mois après un infarctus, ou
- patient atteint d'une cardiomyopathie dilatée en apparence primitive, avec une fraction d'éjection ≤ 35 % et symptômes d'insuffisance cardiaque légère ou modérée (classes NYHA II ou III).

Cependant, lorsque l'on examine après coup le taux d'utilisation effectif (délivrance d'au moins une thérapie) des défibrillateurs qui ont été implantés chez les patients en suivant ces critères, on peut constater que très peu de patients tirent bénéfice de leur défibrillateur ; ainsi, dans une étude de 2005, il apparait que 81 % des patients n'ont jamais fait l'objet d'une délivrance d'une thérapie par le défibrillateur sur une période de 5 ans.

Or l'implantation d'un défibrillateur n'est pas une opération anodine :
- en premier lieu, les algorithmes du défibrillateur peuvent détecter à tort des tachycardies ventriculaires, conduisant à la délivrance de chocs inutiles, voire délétères, qui altèrent la qualité de vie du patient. Au-delà du désagrément dû à ce type de chocs douloureux et angoissants pour le patient, il a également été démontré que ces chocs inappropriés augmentent les risques de décès ;
- en deuxième lieu, les sondes de défibrillation sont des dispositifs complexes et fragiles, avec un taux de défaillance notablement plus élevé que celui des simples sondes de stimulation. Les patients équipés d'un défibrillateur implantable font donc, statistiquement, plus souvent l'objet de réinterventions que ceux dotés d'un simple stimulateur ou resynchroniseur ;
- en troisième lieu, les défibrillateurs implantés en prévention primaire sont souvent programmés de manière à ne traiter que les arythmies à rythme très élevé. Dans la zone des "tachycardies ventriculaires lentes" ("TV lentes"), entre 100 et 150 bpm environ, toutes les thérapies sont désactivées, cette zone n'étant utilisée que pour le suivi du patient. Or ces TV lentes pourraient être stoppées par des thérapies alternatives à la délivrance d'un choc, notamment les thérapies dites "ATP" (*AntiTachycardia Pacing*) agissant par stimulation programmée à une fréquence adaptée à la tachycardie, qui empêcheraient l'état du patient de s'aggraver.

Le but de l'invention est de remédier à ces difficultés en proposant un ensemble permettant l'évaluation et l'adaptation d'une thérapie antitachycardique par délivrance de chocs. Cet ensemble comprend un défibrillateur implantable capable d'évaluer de façon automatique les risques réels d'arythmie ventriculaire délétère chez le patient, et d'adapter automatiquement sa programmation (modification des algorithmes d'analyse de rythme, de la sensibilité de détection, etc.) en fonction du risque réellement encouru par ce patient, afin de ne déclencher une alarme et/ou activer une possible thérapie de choc qu'en cas de risque majeur d'arythmie. Dans le cas contraire, les thérapies de choc pourront être désactivées, évitant ainsi la délivrance de chocs inappropriés, douloureux et délétères, au profit également d'une éventuelle thérapie ATP.

Les techniques d'analyse proposées jusqu'à présent pour quantifier le risque de survenue d'arythmie ventriculaire maligne sont, pour la majeure partie d'entre elles, basées sur l'étude d'un seul descripteur (études univariées) : occurrence de tachycardies ventriculaires non soutenues (NSVT, *Non-Sustained Ventricular Tachycardia*)*,* variabilité du rythme cardiaque (HRV, *Heart Rate Variability*)*,* turbulence du rythme cardiaque (HRT, *Heart Rate Turbulence),* largeur du complexe QRS, longueur du segment QT, etc.

Par exemple, le US 2011/0190650 A1 évalue un risque de mort subite en se basant exclusivement sur la dispersion du segment QT, descripteur qui est mesuré sur des enregistrements ECG et comparé à une valeur préétablie : si la dispersion mesurée est significativement différente de la valeur préétablie, le patient est considéré à risque de mort subite, ce qui justifie l'implantation prophylactique d'un défibrillateur.

Des études multivariées ont également été menées, par exemple pour évaluer la combinaison alternance de l'onde T (TWA, *T-Wave Alternans*) + potentiels tardifs, ou la combinaison HRT + TWA + fraction d'éjection réduite. Ces études multivariées n'évaluent toutefois que des combinaisons linéaires de descripteurs, avec une spécificité guère supérieure à ce qui avait pu être obtenu avec les autres techniques.

Le WO 2009/088627 A1 repose ainsi sur la comparaison de descripteurs obtenus à partir d'un enregistrement Holter à des valeurs prédéterminées, le critère de risque étant basé sur le nombre de descripteurs dépassant les seuls préétablis.

Le US 2011/0301479 A1 prévoit-de calculer tous les descripteurs d'un enregistrement Holter et de les répartir en trois catégories, correspondant à la décomposition du triangle de Coumel (qui sera expliquée plus bas). Dans chacune de ces trois catégories, les descripteurs sont comparés à des valeurs préétablies considérées comme "normales", et pour chaque catégorie un sous-critère de risque est établi en fonction du rapport entre les descripteurs qui se situent dans les bornes de cette "normalité" et ceux qui se situent hors de ces mêmes bornes. Le critère de risque final de mort subite correspond au rapport entre le nombre de ces trois sous-critères de risque catégoriel validés ou non.

Le US 5 251 626 A décrit un dispositif de détection et de traitement des arythmies mettant en oeuvre un réseau de neurones. Cette technique a toutefois pour inconvénient d'analyser globalement les divers paramètres concourant au déclenchement d'une arythmie, en moyennant et en mélangeant les informations des divers critères arythmogènes pertinents. La spécificité du dispositif est de ce fait relativement médiocre, conduisant à une proportion élevée de faux positifs et de faux négatifs.

Les différentes techniques ci-dessus se révèlent ainsi en pratique peu discriminantes car elles reposent sur des modèles trop simples (à descripteur unique et/ou basé sur des franchissements de seuil) ou ne reflètent pas la réalité des phénomènes physiologiques, qui mettent en jeu un nombre important de facteurs interagissant de manière non linéaire. Pour éviter ces inconvénients et atteindre les buts précités, l'invention propose un ensemble comportant, de manière en elle-même connue d'après le US 5 251 626 A précité :
a) un dispositif médical actif apte à être implanté chez un patient, comprenant :
   - des moyens de délivrance de chocs de défibrillation ;
   - des moyens de recueil en continu de paramètres d'activité cardiaque courante du patient ; et
   - des moyens évaluateurs comprenant un réseau de neurones avec au moins deux couches.

De façon caractéristique de l'invention :
- ledit réseau de neurones comprend :
   - en amont, trois sous-réseaux de neurones, recevant les paramètres répartis en trois sous-groupes respectifs distincts correspondant à des classes de facteurs arythmogènes, avec un premier desdits sous-groupes comprenant des descripteurs de substrat électrophysiologique, un deuxième desdits sous-groupes comprenant des descripteurs de modulateurs péjoratifs, et un troisième desdits sous-groupes comprenant des descripteurs de facteur gâchette ;
   - en aval, un réseau de neurones de sortie comprenant au moins un neurone de sortie couplé aux trois sous-réseaux amont et apte à délivrer un indice de risque d'arythmie ventriculaire, et
- le dispositif comprend en outre des moyens aptes à comparer l'indice de risque d'arythmie ventriculaire à un seuil donné et à activer ou désactiver au moins une fonction du dispositif en cas de franchissement du seuil.

La fonction du dispositif activée ou désactivée en cas de franchissement dudit seuil peut notamment être une fonction parmi : production d'une alarme ; activation/désactivation des thérapies par choc de défibrillation ; activation de nouvelles zones de thérapie ; modification de la sensibilité du détecteur d'arythmie ; activation/désactivation d'algorithmes ; et/ou modification de paramètres de thérapies.

Pour sa mise en oeuvre, l'ensemble ci-dessus comprend en outre :
b) une base de données de patients de référence, mémorisant pour chaque patient de référence :
   - une pluralité de descripteurs élaborés à partir des paramètres d'activité cardiaque recueillis pour le patient de référence, et
   - un marqueur indiquant la survenue avérée, ou non, d'une arythmie ventriculaire chez le patient de référence, et
c) des moyens aptes à définir la structure du réseau de neurones par apprentissage supervisé à partir de la base de données de patients de référence, comprenant :
   - pour chacun des sous-groupes correspondant à des classes de facteurs arythmogènes :
      - des moyens de sélection des descripteurs les plus pertinents ;
      - des moyens de détermination de la structure des sous-réseaux de neurones ; et
      - des moyens d'optimisation des sous-réseaux de neurones, et
   - des moyens de construction du réseau de neurones ; et
   - des moyens d'apprentissage du réseau de neurones.

Le premier sous-groupe peut notamment comprendre des descripteurs de substrat électrophysiologique choisis parmi : QRS residuum et/ou T-Wave residuum ; angle QRS-T ; intervalles QTₐₚₑₓ et/ou QT_{end} ; pente descendante de l'onde T ; et/ou décalage du segment ST.

Le deuxième sous-groupe peut notamment comprendre des descripteurs de modulateurs péjoratifs choisis parmi : turbulence du rythme cardiaque ; indice de variabilité entre complexes successifs ; écart-type des moyennes des intervalles normaux ; et/ou représentation de Poincaré de la variabilité du rythme cardiaque.

Le troisième sous-groupe peut notamment comprendre des descripteurs de facteur gâchette choisis parmi : épisode de bigéminisme ou de trigéminisme ventriculaire ; tachycardie ventriculaire ; et/ou extrasystole supraventriculaire.

On va maintenant décrire un exemple de mise en oeuvre de l'invention, en référence aux dessins annexés où les mêmes références désignent d'une figure à l'autre des éléments identiques ou fonctionnellement semblables.
La Figure 1 représente de façon schématique les différents éléments impliqués dans la mise en oeuvre de l'invention, avec un défibrillateur implanté chez un patient.
La Figure 2 illustre schématiquement le fonctionnement de l'évaluateur de risque d'arythmie mis en oeuvre au sein du défibrillateur implanté chez le patient.
La Figure 3 est un exemple de réseau de neurones utilisable pour la mise en oeuvre de l'évaluateur de risque d'arythmie selon l'invention.
La Figure 4 est un organigramme explicitant les différentes étapes de la construction et de l'apprentissage du réseau de neurones utilisé pour la mise en oeuvre de l'invention.

On va maintenant décrire un exemple de réalisation de l'invention.

En ce qui concerne ses aspects logiciels, l'invention peut être mise en oeuvre au sein d'un dispositif médical implantable connu par une programmation appropriée du logiciel de commande de celui-ci.

L'invention peut notamment être appliquée aux dispositifs implantables tels que ceux de la famille *Paradym* produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation et de défibrillation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

L'invention est mise en oeuvre par des moyens principalement logiciels, au moyen d'algorithmes appropriés exécutés par un microcontrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées par un même logiciel.

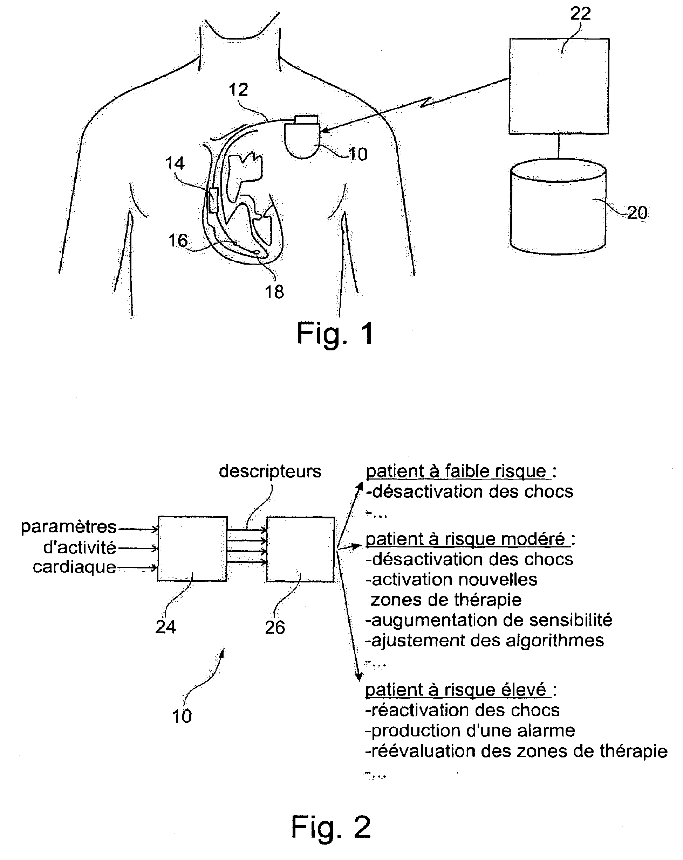

Sur la Figure 1, la référence 10 désigne un implant cardiaque de type défibrillateur ou stimulateur/défibrillateur, pourvu d'une sonde 12 munie d'un bobinage 14 formant électrode de défibrillation, ainsi que d'une pluralité d'électrodes ou capteurs 16, 18, permettant le recueil de divers signaux d'activité cardiaque tels qu'électrogramme endocavitaire (EGM) et signal d'accélération endocardiaque (EA) notamment. Le dispositif 10 peut également être pourvu de divers moyens de recueil de signaux reflétant l'activité métabolique du patient (bioimpédance intracardiaque et/ou transpulmonaire, ventilation-minute, etc.) ou encore d'activité physique du patient (capteur d'accélération). Ces divers signaux seront après désignés collectivement sous le terme de "paramètres d'activité cardiaque".

De façon caractéristique, pour la mise en oeuvre de l'invention, l'ensemble comprend également une base de données 20 de patients de référence mémorisant, pour une population de patients ayant fait l'objet antérieurement d'un suivi, diverses informations dérivées des paramètres d'activité cardiaque recueillis pour chacun de ces patients, ainsi que, pour chacun d'entre eux, un marqueur ou étiquette indiquant qu'une arythmie ventriculaire maligne a été, ou non, détectée pendant une période de suivi prédéterminée.

Cette base de données est utilisée par un module 22 permettant de définir la structure optimale, pour le problème étudié, d'un classifieur automatique et de réaliser l'apprentissage supervisé dudit classifieur, ce dernier comportant, selon l'invention, un réseau de neurones. Le modèle ainsi conçu sera programmé dans le dispositif implanté 10 de manière que celui-ci puisse évaluer en continu, au moyen de ce classifieur, le risque d'arythmie du patient porteur de l'implant.

La Figure 2 illustre schématiquement le principe de fonctionnement de cet évaluateur de risque d'arythmie tel qu'il est mis en oeuvre au sein du dispositif implanté 10.

Les divers paramètres d'activité cardiaque recueillis par le dispositif implanté font tout d'abord l'objet d'un premier traitement (bloc 24) permettant d'extraire un certain nombre de "descripteurs". Ces descripteurs sont des données, calculées à partir des paramètres d'activité cardiaque, présentant une pertinence pour l'évaluation du risque d'arythmie. Divers exemples de tels descripteurs seront présentés ci-après.

Ces descripteurs sont appliqués en entrée à un module d'estimation du risque d'arythmie (bloc 26), qui comprend des moyens permettant d'obtenir en sortie, à partir de la multiplicité de descripteurs appliqués en entrée, un indice unique permettant de quantifier, pour le patient implanté, le risque courant d'arythmie ventriculaire maligne.

L'indice ainsi obtenu est comparé à un seuil prédéterminé, le patient étant alors qualifié de patient "à faible risque" ou "à risque élevé" selon le cas. Le franchissement du seuil, dans un sens ou dans l'autre, a pour effet de modifier une ou plusieurs fonctions du dispositif, par exemple :
- pour un patient devenant "à risque élevé" : émission d'une alarme ; activation ou réactivation des thérapies par choc de défibrillation ;
- pour un patient devenant "à risque modéré" : désactivation des thérapies par choc de défibrillation ; activation de nouvelles zones de thérapie, de manière à traiter non seulement les arythmies à rythme élevé, mais également les "tachycardies ventriculaires lentes" à rythme compris entre 100 et 150 bpm ; augmentation de la sensibilité du détecteur d'arythmies, par exemple avec un seuil d'amplitude des ondes détectées réglé à 0,4 mV ; désactivation de certains algorithmes ; et/ou ajustement des paramètres des thérapies ;
- pour un patient devenant "à faible risque" : désactivation des thérapies par chocs de défibrillation, pour réduire les risques de chocs inappropriés, délétères, en n'activant que les thérapies de type ATP ; abaissement de la sensibilité du détecteur d'arythmies ; activation/désactivation de certains algorithmes, etc.

Les descripteurs susceptibles d'être utilisés pour évaluer l'indice de risques d'arythmie sont répartis en trois groupes. La décomposition en trois groupes permet de transcrire mathématiquement une approche physiologique du problème, schématisée par le "triangle de Coumel". Pour se développer et se maintenir, une arythmie requiert la coexistence de trois facteurs, à savoir :
- un terrain structurel ou fonctionnel pathologique (sommet "substrat électrophysiologique" du triangle) tel qu'une zone à conduction lente du myocarde, des antécédents d'infarctus, une pathologie affectant les caractéristiques électrophysiologiques des cellules, etc. ;
- un évènement déclenchant (sommet "facteur gâchette" du triangle) : augmentation de la fréquence cardiaque, extrasystole, etc. ; et
- un environnement favorable à la pérennisation de cette arythmie (sommet "modulateurs péjoratifs" du triangle) : baisse de la variabilité sinusale, hypokaliémie, etc.

Dans le cas présent, le choix du groupe auquel est affecté chaque descripteur est motivé par la nature du phénomène physiologique pour lequel ce paramètre est pertinent.

En ce qui concerne le groupe de descripteurs "substrat électrophysiologique", celui-ci peut comprendre (cette liste n'étant bien entendu ni exhaustive ni limitative) :
- les marqueurs QRS Residuum et T-Wave Residuum, qui sont, respectivement, des marqueurs de l'hétérogénéité des dépolarisations et repolarisations ventriculaires. Ces descripteurs peuvent être obtenus par une analyse en composantes principales des signaux correspondant au complexe QRS d'une part, et à l'onde T d'autre part ;
- l'angle QRS-T, c'est-à-dire l'angle compris entre l'axe de la dépolarisation ventriculaire et celui de la repolarisation ventriculaire. Ces axes sont calculés par analyse en composantes principales d'un signal EGM ;
- les intervalles QTₐₚₑₓ et QT_{end}, qui sont les intervalles compris, respectivement, entre le début du complexe QRS et le sommet ou la fin de l'onde T. Ces intervalles sont évalués à partir d'un enregistrement de signal EGM et peuvent faire l'objet d'une correction en fonction du rythme cardiaque instantané ;
- la pente descendante de l'onde T, correspondant au coefficient directeur de la plus raide des régressions linéaires calculées sur trois points consécutifs à partir du sommet de l'onde T ;
- le décalage du segment ST, mesuré au niveau du point "J" (60 ms après la fin du QRS), par rapport à la ligne isoélectrique.

En ce qui concerne le groupe de descripteurs "facteurs gâchette", celui-ci peut comprendre la survenue d'épisodes tels que (cette liste n'étant bien entendu ni exhaustive ni limitative) :
- bigéminisme ou trigéminisme ventriculaire ;
- tachycardie ventriculaire ;
- extrasystole supraventriculaire ;
- doublet supraventriculaire.

En ce qui concerne le groupe de descripteurs "modulateurs péjoratifs", celui-ci peut comprendre (cette liste n'étant bien entendu ni exhaustive ni limitative) :
- la turbulence du rythme cardiaque (HRT, *Heart Rate Turbulence),* caractérisée par les valeurs TO (*Turbulence Onset,* survenue de la turbulence) et TS (*Turbulence Slope,* pente de la turbulence) ;
- l'indice de variabilité, défini comme le pourcentage des écarts moyens entre deux QRS successifs ;
- la valeur SDANN (*Standard Deviation of Averages of All Normal-to-Normal Intervals,* écart-type des moyennes de tous les intervalles normal-normal) ;
- le "Poincaré Plot SD2", qui est une représentation en deux dimensions des intervalles RR en fonction des intervalles RR les précédant, approchée par une ellipse dont le grand axe et le petit axe constituent deux descripteurs représentatifs.

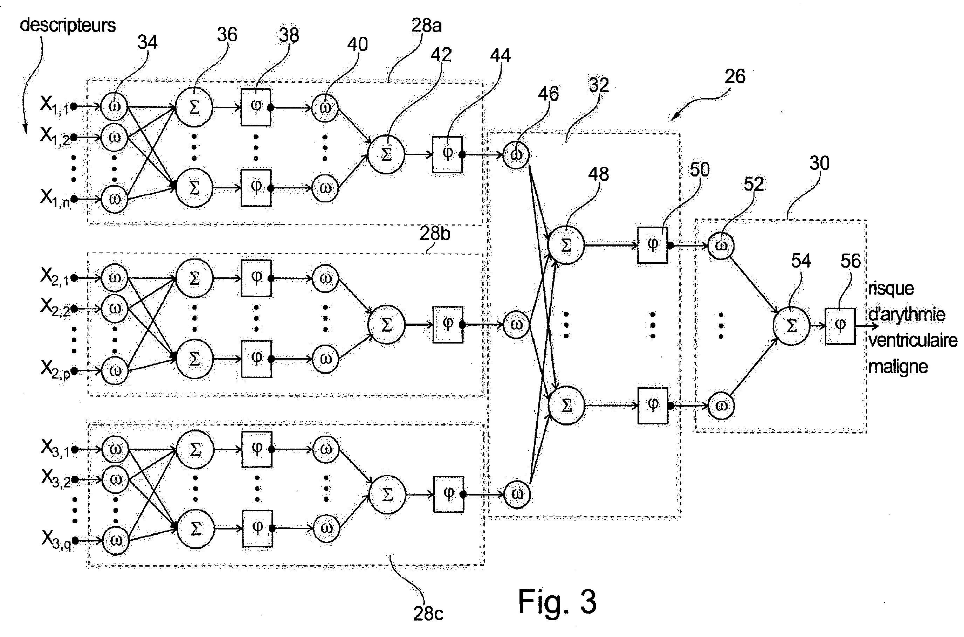

La Figure 3 illustre un exemple de réseau de neurones 26 recevant en entrée les descripteurs que l'on vient d'indiquer, et produisant en sortie un indice représentatif du risque d'arythmie ventriculaire maligne.

De façon caractéristique de l'invention, ce réseau 26 comprend en amont trois sous-réseaux distincts 28a, 28b et 28c recevant les descripteurs respectifs correspondant à chacun des trois groupes décrits ci-dessus (substrat électrophysiologique, facteur gâchette et modulateurs péjoratifs), ces trois sous-réseaux étant connectés, côté aval, à un neurone de sortie 30 délivrant en sortie l'indice représentatif du risque d'arythmie ventriculaire. Dans l'exemple illustré, les réseaux amont 28a, 28b et 28c sont connectés au neurone de sortie 30 via une couche cachée 32, mais cet exemple n'est pas limitatif, les sous-réseaux 28a, 28b, 28c pouvant être directement connectés au neurone de sortie 30.

La représentation de la Figure 3 est la représentation canonique d'un réseau de neurones selon un mode de réalisation de l'invention, avec en 34 les différents poids ω appliqués respectivement à chacune des entrées (descripteurs) X_{1,1} ... X_{1,n} du sous-réseau 28a, des entrées X_{2,1} ... X_{2,p} du deuxième sous-réseau 28b et des entrées X_{3,1} ... X_{3,q} du troisième sous-réseau 28c.

Les neurones cachés de chacun des sous-réseaux 28a, 28b, 28c réalisent en 36 une sommation pondérée des différentes entrées puis appliquent en 38 une fonction φ d'activation des neurones cachés. Les sorties obtenues sont pondérées en 40 puis font l'objet en 42 d'une sommation et en 44 de la fonction d'activation de sortie du sous-réseau.

Chacune des sorties respectives des sous-réseaux 28a, 28b, 28c fait l'objet en 46 d'une pondération par un poids ω pour sommation en 48 et application de fonction d'activation 50 des neurones cachés de la couche cachée 32. Les sorties de cette couche cachée font elles-mêmes l'objet d'une pondération en 52 par des poids respectifs ω pour le neurone de sortie 30, qui assure la sommation en 54 et l'application de la fonction d'activation finale en 56.

Dans une configuration simplifiée chacun des sous-réseaux 28a, 28b, 28c pourrait toutefois être constitué d'un seul neurone, avec la sortie de chaque sous-réseau 28a, 28b, 28c reliée au neurone de sortie 30, sans couche cachée ni neurones cachés.

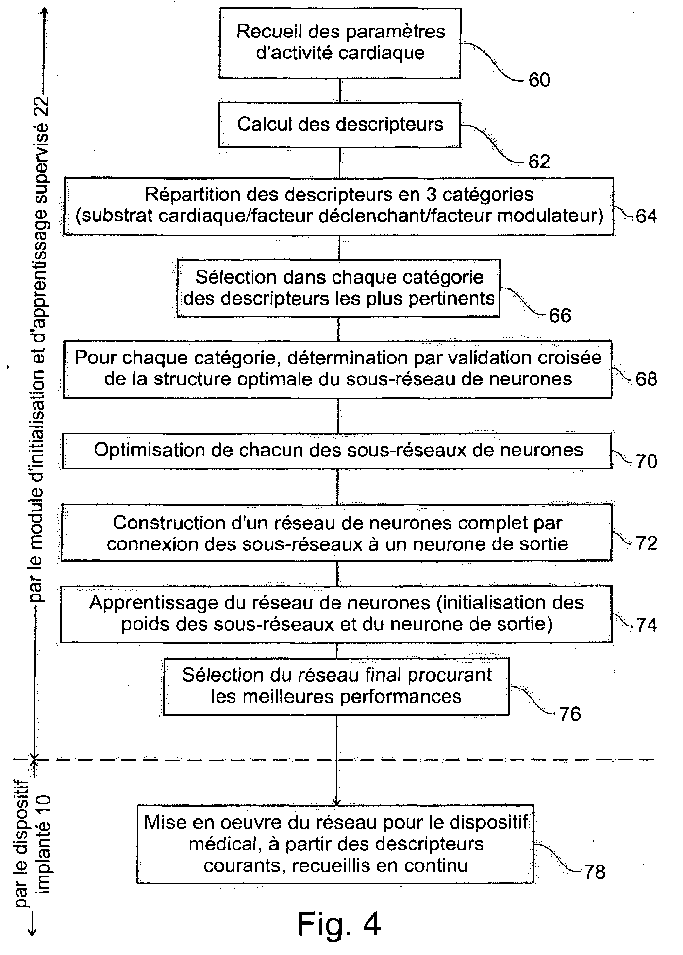

La Figure 4 est un organigramme explicitant les différentes étapes de la construction et de l'apprentissage du réseau de neurones que l'on vient de décrire.

Les paramètres d'activité cardiaque sont recueillis pour une population de patients de référence (étape 60) et les descripteurs correspondants sont calculés et mémorisés dans la base de données 20 (étape 62).

Ces patients de référence sont des patients porteurs d'un défibrillateur en prévention primaire, et font l'objet d'un suivi sur une durée longue, typiquement d'au moins six mois. La base de données est étiquetée, c'est-à-dire que l'on sait pour chaque patient s'il a ou non subi une thérapie par au moins un choc de défibrillation délivré par son implant, sur la période considérée.

Les descripteurs qui ont été calculés sont répartis (étape 64) en trois catégories correspondant aux trois facteurs arythmogènes à l'origine d'une arythmie (substrat électrophysiologique, facteur gâchette et modulateurs péjoratifs), comme schématisé par le principe du triangle de Coumel. Cette répartition des descripteurs en trois groupes traduit des connaissances physiologiques et, d'autre part, permet de limiter la complexité du réseau de neurones final.

L'étape suivante (étape 66) est une sélection dans chaque catégorie des descripteurs les plus pertinents, par exemple par un traitement de type orthogonalisation de Gram-Schmidt avec vecteur sonde, qui permet de classer dans chaque catégorie les descripteurs en fonction de leur capacité à "expliquer" l'étiquette des patients, c'est-à-dire l'existence ou non d'une thérapie appropriée dans la période considérée. Ceci permet d'éliminer éventuellement un ou plusieurs descripteurs qui ne seraient pas pertinents, ou pas suffisamment pertinents pour le but recherché.

Pour chaque catégorie de descripteur, la structure optimale du réseau de neurones (c'est-à-dire de chacun des sous-réseaux 28a, 28b, 28c de la Figure 3) recevant en entrée les descripteurs de la catégorie considérée est déterminée, par validation croisée selon une technique en elle-même connue (étape 68). Cette détermination de la structure des sous-réseaux 28a, 28b, 28c a pour objet de déterminer leur nombre de neurones cachés, de manière que le réseau soit en mesure de reproduire ce qui est déterministe dans les données qui lui sont appliquées. On notera que la décomposition des descripteurs en trois sous-groupes distincts permet, pour un même nombre de neurones et d'entrées, de réduire très fortement le nombre de paramètres à ajuster pour déterminer l'architecture du réseau.

Les différents sous-réseaux sont ensuite optimisés (étape 70) puis connectés à un neurone de sortie (le neurone 30 de la Figure 3), avec ou sans couche cachée intermédiaire (étape 72).

Le réseau final ainsi constitué fait l'objet d'apprentissages multiples (étape 74) en initialisant les poids ω des trois sous-réseaux 28a, 28b, 28c conformément au réseau déterminé précédemment, et en initialisant les poids ω du neurone de sortie de manière aléatoire (étape 74).

Le réseau final procurant les meilleures performances est alors sélectionné (étape 76), et il peut alors être programmé dans le logiciel de commande du dispositif implanté 10.

Le réseau sera alors mis en oeuvre par le dispositif 10 de la manière expliquée plus haut en référence à la Figure 2, en continu, à partir des descripteurs courants calculés à partir des divers paramètres d'activité cardiaque recueillis chez le patient porteur du dispositif 10 (étape 78).

Par ailleurs, le réseau peut être régulièrement mis à jour. Cette mise à jour peut être effectuée à l'aide d'un ordinateur, d'une tablette, d'un enregistreur d'évènements tel que le modèle *Spiderflash* produit et commercialisé par Sorin CRM, Clamart, France, qui peut être synchronisé avec l'implant pour la mise à jour.

## Revendications

1. Un ensemble pour l'évaluation et l'adaptation d'une thérapie antitachycardique, comprenant:
a) un dispositif médical actif (10) apte à être implanté chez un patient, comprenant :
- des moyens de délivrance de chocs de défibrillation ;
- des moyens de recueil en continu (24) de paramètres d'activité cardiaque courante du patient ; et
- des moyens évaluateurs (26) comprenant un réseau de neurones avec au moins deux couches,
**caractérisé en ce que** ledit réseau de neurones comprend :
• en amont, trois sous-réseaux de neurones (28a, 28b, 28c), recevant les paramètres répartis en trois sous-groupes respectifs distincts correspondant à des classes de facteurs arythmogènes, avec :
un premier desdits sous-groupes comprenant des descripteurs de substrat électrophysiologique,
un deuxième desdits sous-groupes comprenant des descripteurs de modulateurs péjoratifs, et
un troisième desdits sous-groupes comprenant des descripteurs de facteur gâchette ;
• en aval, un réseau de neurones de sortie comprenant au moins un neurone de sortie (30) couplé aux trois sous-réseaux amont et apte à délivrer un indice de risque d'arythmie ventriculaire,
et **en ce que** le dispositif médical actif comprend en outre :
- des moyens aptes à comparer l'indice de risque d'arythmie ventriculaire à un seuil donné et à activer ou désactiver au moins une fonction du dispositif en cas de franchissement du seuil.

2. L'ensemble de la revendication 1, dans lequel la fonction du dispositif activée ou désactivée en cas de franchissement dudit seuil est une fonction parmi : production d'une alarme ; activation/désactivation des thérapies par choc de défibrillation ; activation de nouvelles zones de thérapie ; modification de la sensibilité du détecteur d'arythmie ; activation/désactivation d'algorithmes ; et/ou modification de paramètres de thérapies.

3. L'ensemble de la revendication 1, comprenant en outre :
b) une base de données (20) de patients de référence, mémorisant pour chaque patient de référence :
- une pluralité de descripteurs élaborés à partir des paramètres d'activité cardiaque recueillis pour le patient de référence, et
- un marqueur indiquant la survenue avérée, ou non, d'une arythmie ventriculaire chez le patient de référence, et
c) des moyens (22) aptes à définir la structure du réseau de neurones par apprentissage supervisé à partir de la base de données de patients de référence, comprenant :
- pour chacun des sous-groupes correspondant à des classes de facteurs arythmogènes :
• des moyens de sélection des descripteurs les plus pertinents ;
• des moyens de détermination de la structure des sous-réseaux de neurones ; et
• des moyens d'optimisation des sous-réseaux de neurones, et
- des moyens de construction du réseau de neurones ; et
- des moyens d'apprentissage du réseau de neurones.

4. L'ensemble de la revendication 3, dans lequel ledit premier des sous-groupes comprend des descripteurs de substrat électrophysiologique choisis parmi : QRS residuum et/ou T-Wave residuum ; angle QRS-T ; intervalles QTₐₚₑₓ et/ou QT_{end} ; pente descendante de l'onde T ; et/ou décalage du segment ST.

5. L'ensemble de la revendication 3, dans lequel ledit deuxième des sous-groupes comprend des descripteurs de modulateurs péjoratifs choisis parmi : turbulence du rythme cardiaque ; indice de variabilité entre complexes successifs ; écart-type des moyennes des intervalles normaux ; et/ou représentation de Poincaré de la variabilité du rythme cardiaque.

6. L'ensemble de la revendication 3, dans lequel ledit troisième des sous-groupes comprend des descripteurs de facteur gâchette choisis parmi : épisode de bigéminisme ou de trigéminisme ventriculaire ; tachycardie ventriculaire ; et/ou extrasystole supraventriculaire.

## Patentansprüche

1. Anordnung zum Bewerten und Anpassen einer Antitachykardie-Therapie, die Folgendes umfasst:
a) eine aktive medizinische Vorrichtung (10), die in einen Patienten implantiert werden kann und enthält:
- Mittel zum Verabreichen von Defibrillationsstößen;
- Mittel (24) zum kontinuierlichen Sammeln von aktuellen Herzaktivitätsparametern des Patienten; und
- Bewertungsmittel (26), die ein Neuronennetz mit wenigstens zwei Schichten enthalten,
**dadurch gekennzeichnet, dass** das Neuronennetz Folgendes umfasst:
• stromaufseitig drei Unterneuronennetze (28a, 28b, 28c), die die Parameter empfangen, die auf drei verschiedene jeweilige Untergruppen, die Klassen arrhythmogener Faktoren entsprechen, verteilt sind, mit:
einer ersten Untergruppe, die Deskriptoren eines elektrophysiologischen Substrats enthält,
einer zweiten Untergruppe, die Deskriptoren von pejorativen Modulatoren enthält, und
einer dritten Untergruppe, die Deskriptoren eines Auslösefaktors enthält;
• stromabseitig ein Ausgangsneuronennetz, das wenigstens ein Ausgangsneuron (30) enthält, das mit den drei stromaufseitigen Unternetzen gekoppelt ist und eine Angabe eines ventrikulären Arrhythmierisikos liefern kann,
und dass die aktive medizinische Vorrichtung außerdem Folgendes umfasst:
- Mittel, die die Angabe des ventrikulären Arrhythmierisikos mit einem gegebenen Schwellenwert vergleichen können und wenigstens eine Funktion der Vorrichtung im Fall eines Durchgangs durch den Schwellenwert aktivieren oder deaktivieren können.

2. Anordnung nach Anspruch 1, wobei die Funktion der aktivierten oder nicht aktivierten Vorrichtung im Fall eines Durchgangs durch den Schwellenwert eine der folgenden Funktionen ist: Erzeugen eines Alarms; Aktivieren/Deaktivieren von Therapien durch Defibrillationsstoß; Aktivieren neuer Therapiezonen; Modifizieren der Empfindlichkeit des Arrhythmie-Detektors; Aktivieren/Deaktivieren von Algorithmen; und/oder Modifizieren von Therapieparametern.

3. Anordnung nach Anspruch 1, die außerdem Folgendes umfasst:
b) eine Datenbank (20) von Referenzpatienten, die für jeden Referenzpatienten Folgendes speichert:
- mehrere Deskriptoren, die anhand von Herzaktivitätsparametern, die für den Referenzpatienten gesammelt wurden, entwickelt worden sind, und
- eine Markierung, die das erwiesene oder nicht erwiesene Auftreten einer ventrikulären Arrhythmie bei dem Referenzpatienten angibt, und
c) Mittel (22), die die Struktur des Neuronennetzes durch überwachtes Lernen anhand der Datenbank von Referenzpatienten definieren können und Folgendes umfassen:
- für jede der Untergruppen, die den Klassen arrhythmogener Faktoren entsprechen:
• Mittel zum Auswählen der treffendsten Deskriptoren;
• Mittel zum Bestimmen der Struktur der Neuronenunternetze; und
• Mittel zum Optimieren der Neuronenunternetze und
- Mittel zum Konstruieren des Neuronennetzes; und
- Mittel zum Lernen des Neuronennetzes.

4. Anordnung nach Anspruch 3, wobei die erste der Untergruppen Deskriptoren eines elektrophysiologischen Substrats umfasst, die gewählt sind aus: QRS-Rest und/oder T-Wellen-Rest; QRS-T-Winkel; QTₐₚₑₓ-Intervalle und/oder QT_{end}-Intervalle, Abstiegsflanke der T-Welle und/oder Versatz des ST-Segments.

5. Anordnung nach Anspruch 3, wobei die zweite der Untergruppen Deskriptoren pejorativer Modulatoren umfasst, die gewählt sind aus: Turbulenz des Herzrhythmus; Angabe der Variabilität zwischen aufeinander folgenden Komplexen; Abstandstyp von Mittelwerten normaler Intervalle; und/oder Poincaré-Darstellung der Variabilität des Herzrhythmus.

6. Anordnung nach Anspruch 3, wobei die dritte der Untergruppen Deskriptoren eines Auslösefaktors umfasst, die gewählt sind aus: Episode des ventrikulären Bigeminus oder Trigeminus; ventrikuläre Tachykardie und/oder supraventrikuläre Extrasystole.

## Claims

1. A unit for evaluation and adaptation of an anti-tachycardia therapy, comprising:
a) an active medical device (10) adapted to be implanted in a patient, comprising:
- means for delivering defibrillation shocks;
- means (24) for continuously collecting parameters of current cardiac activity of the patient; and
- evaluator means (26) comprising a neural network with at least two layers,
**characterized in that** said neural network comprises:
• upstream, three neural subnetworks (28a, 28b, 28c), receiving the parameters distributed into three respective distinct subgroups corresponding to classes of arrhythmogenic factors, with:
a first of said subgroups comprising electrophysiologic substrate descriptors,
a second of said subgroups comprising pejorative modulator descriptors, and
a third of said subgroups comprising trigger factor descriptors;
• downstream, an output neural network comprising at least one output neuron (30) coupled to three upstream subnetworks and adapted to deliver a ventricular arrhythmia risk index,
and **in that** the active medical device further comprises:
- means adapted to compare the ventricular arrhythmia risk index with a given threshold and to activate or deactivate at least one function of the device in case of threshold crossing.

2. The unit of claim 1, wherein the device function activated or deactivated in case of crossing of said threshold is a function among: alarm production; activation/deactivation of the defibrillation shock therapies; activation of new therapy areas; modification of the arrhythmia detector sensitivity; activation/deactivation of algorithms; and/or modification of parameters of therapies.

3. The unit of claim 1, further comprising:
b) a reference patient database (20), storing for each reference patient:
- a plurality of descriptors elaborated based on the parameters of cardiac activity collected for the reference patient, and
- a marker indicating the occurrence, recognized or not, of a ventricular arrhythmia in the reference patient, and
c) means (22) adapted to define the structure of the neural network by supervised learning from the reference patient database, comprising:
- for each of the subgroups corresponding to classes of arrhythmogenic factors:
• means for selecting the most pertinent descriptors;
• means for determining the structure of the neural subnetworks; and
• means for optimizing the neural subnetworks, and
- means for constructing the neural network; and
- means for learning the neural network.

4. The unit of claim 3, wherein said first one of the subgroups comprises electrophysiologic substrate descriptors chosen among: QRS residuum and/or T-wave residuum; QRS-T angle; QTₐₚₑₓ and/or QT_{end} intervals; descending slope of the T-wave; and/or ST segment offset.

5. The unit of claim 3, wherein said second one of the subgroups comprises pejorative modulator descriptors chosen among: cardiac rhythm turbulence; index of variability between successive complexes; standard deviation of the normal intervals averages; and/or Poincarré representation of the cardiac rhythm variability.

6. The unit of claim 3, wherein said third one of the subgroups comprises trigger factor descriptors chosen among: episode of ventricular bigeminy or trigeminy; ventricular tachycardia; and/or supraventricular extrasystole.
